# EUROPEAN PATENT APPLICATION

(11) **EP 1 156 122 A2**
(43) Date of publication of application: **21.11.2001**
(21) Application number: 01118580.8
(22) Date of filing: 05.03.1999
(51) Int. Cl.: C12Q 1/48

(54) **A fluorescence polarization assay method**

(30) Priority: 06.03.1998 DK 30898
(62) Divisional of application: 99906092.4
(71) Applicant: Novozymes A/S, 2880 Bagsvaerd (DK)
(72) Inventor: Kongsbak, Lars, 2840 Holte (DK); Valbjoern, Jesper, 2000 Frederiksberg C (DK); Joergensen, Kristian, Skovgaard, 2200 Koebenhavn N (DK); Joergensen, Christel, Thea, 2100 Koebenhavn O (DK); Husum, Tommy, Lykke, 3400 Hilleroed (DK); Moeller, Soeren, 2840 Holte (DK); Ernst, Steffen, 2200 Koebenhavn N (DK)

(57) **Abstract**

A method for determination of transferase activity is disclosed. The method comprises measuring a change in fluorescence polarization of a fluorescently labeled substance upon transfer to a second compound by the transferase.

## Description

### FIELD OF THE INVENTION

The present invention is a divisional application of parent application EP 99 906 092.4. This invention relates to a method for determination of transferase activity comprising measuring changes in fluorescence polarization of a fluorescently labeled substance upon transfer to a second compound by the transferase. Such a method is useful when screening for a nucleotide sequence in a gene expression system, e.g. a gene library or an *in vitro* expression system encoding for a biological compound. The method exploits the features of fluorescence polarization (FP) for detecting the biological compound generated in the expression system upon reaction with a fluorescent substance. Fluorescence assays are very sensitive, and thus suitable for detecting minute amounts of a biological compound. However using a fluorescence polarization assay is particularly useful as this technique enables sensitive detection as well as robustness against signal scattering or quenching. Also using fluorescence polarization enables homogenous screening assays, i.e. it is not necessary to separate the products from the reaction between the biological compound and the fluorescent substance and unreacted fluorescent substance.

### BACKGROUND OF THE INVENTION

The phenomenon fluorescence polarization (denoted FP) has been known since 1926 when F. Perrin observed that if a fluorescent molecule is excitated by polarized light, the subsequently emitted light will also be polarized in a fixed plane if the molecule remain stationary between excitation and emmison (F. Perrin: *"Polarisation de la lumiere de fluorescence. Vie moyene des molecules dans L'etat exite",* Le Journal de Physique et le Radium, Tome VII, Serie VI, 1926, pgs. 390-401).

Until the late seventies, this property of fluorescent molecules was not paid much attention, probably because of limited applicability due to lack of sufficiently technically refined equipment able of measuring the emitted polarized light and changes in polarization therein.

In 1979 H. Maeda demonstrated an assay for monitoring a protease degradation of large fluorescently labelled proteins using FP technology (Maeda H., Anal. Biochem. 92, 222-227 (1979)) while similar degradative assays for DNA and RNA were presented later (Bolger R. & Checovich W., BioTechniques, 17, 585-589, 1994; Yonemura K. & Maeda H., J. Biochem., 92, 1297-1303, 1982).

The most extensive use of FP has been in the area of clinical immunological chemistry where a number of various assays for determination of specific biological compounds has been developed based on the binding of the compound to a fluorescently labeled antibody the reaction creating a change in the polarization (Checovich W., Bolger R. & Burke T., *Fluorescence polarization - a new tool for cell and molecular biology,* Nature, 375, 254-256, 1995). This reference also describes the characteristic elements of the FP technology.

EP 194472 B1 describes a method for measuring hydrolase activity by measuring the change in fluorescence polarization upon degradation of the substrate by the hydrolase.

Schade, S.Z. et al describes an assay wherein activity of surface associated proteases is detected on spirochetes bacteria, thereby reflecting the pathological potential of the bacteria (Schade, S.Z., Jolley M., Sarauer B.J., Simonson L.G., *Spirochete protease detection by fluorescence polarization,* Journal of Dental Research, 73, pp. 248-248, 1994.

WO 93/11249 describes screening procedures wherein interesting nucleotide sequences encoding for enzymes are transformed into host cells and are identified by measuring clearing zones in substrate agars or gels.

WO 98/58085 (published on 23.12.98 after the priority date of this application) describes a method for screening a prokaryotic expression library for bioactivities (i.e. enzymes) by generating the expression library, inserting, e.g. by staining, a fluorescent substrate in the cells. Enzymes produced by the cells may react with the substrate and generate a fluorescence signal, which on a single cell basis is used to separate positive cells in FACS (Fluorescence Activated Cell Sorter) machine. In order for this technique to work, it is required that the substrate may be constrained within the cellular entity, which in turn means that the substrate must possess properties enabling insertion into a cell. The document discloses accordingly only examples on one type of substrate (C₁₂-FDG) for which a successful insertion into a viable cell has been achieved. Further the potential substrates mentioned in this document are synthetic substrates designed to cause a change in intensity upon reaction with an enzyme, i.e. measurement of intensity changes being the basic principle in this disclosure

Also preparation of a gene library by extraction of DNA from a nucleotide source and preparation and purification of specific fragments of this DNA or copies thereof is known in the art as well as techniques for insertion of these fragments or nucleotide sequences into a host cell (e.g. by ligation in plasmids subsequently transformed into host cells) and expression of such fragments. Expression of nucleotide sequences in *in vitro* systems is also known to the art.

### SUMMARY OF THE INVENTION

Most often screening for nucleotide sequences encoding a biological compound of interest requires contacting the biological compound with a substance which will undergo a detectable change upon reaction with the biological compound. The skilled person will usually have a range of such substances to choose from, but there is a desire to choose substances which resembles substances with which the biological compound will react in an intended real life industrial application. In the case of biological compounds such as enzymes, in choosing a real type substrate to which an interesting enzyme has a high specificity in the screening process one advantage is that new enzymes found in the screening process also are very likely to work well in the intended industrial application. Choosing e.g. a low molecular synthetic substrate of low specificity instead, however, may generate a large number of false positive hits in the screening, i.e. enzymes may be found which reacts well with the synthetic substrate, but will perform poorly on the real substrate in the intended industrial application. The prior art does not teach how to achieve such an advantage in a high throughput screening process for nucleotide sequences.

We have however found a new and versatile use of the fluorescence polarization technology which provides a fast, sensitive and accurate mean for screening nucleotide sources for nucleotide sequences encoding a biological compound suitable for industrial production and application such as an enzyme or a medical drug. Measurements of fluorescence polarization of a fluorescent molecule in stead of changes in the emission intensity of the fluorescent molecule provides a large degree of freedom in choosing the type fluorescent molecule. Also using fluorescence polarization provides finding of considerably more nucleotide sequences encoding relevant biological compounds than is obtainable from methods described in prior art such visual detection of clearing zones around colonies of microorganisms plated out on an agar plate.

In a first aspect the invention provides: A method for screening for a nucleotide sequence encoding for a biological compound comprising:
a) expressing the nucleotide sequence in an expression system, so as to produce the biological compound,
b) contacting the biological compound with a fluorescent substance capable of reacting with the biological compound so as to let the reaction take place,
c) measuring the fluorescence polarization of the fluorescent substance, and
d) selecting expression systems for which a change in fluorescence polarization has occurred.

The invention also in turn provides: A method for producing a biological compound comprising,
a) identifying the biological compound and the nucleotide sequence encoding the biological compound in the context of the invention,
b) culturing a microorganism comprising the identified nucleotide sequence,
c) recovering the biological compound and
d) optionally formulating the biological compound as a liquid or dry product.

In further aspects the invention encompasses specific fluorescence polarization assay methods suitable for use in the screening procedure. Accordingly the invention provides specific methods for:
detection of xylanase activity comprising measuring changes in fluorescence polarization of a fluorescently labeled xylan upon hydrolysis by said xylanase.
detection of pectinase activity comprising measuring changes in fluorescence polarization of a fluorescently labeled pectin upon degradation by said pectinase.
detection of amylase activity comprising measuring changes in fluorescence polarization of a fluorescently labeled starch, limit dextrins, amylose or amylopectin upon hydrolysis by said amylase.
detection of transglutaminase activity comprising measuring changes in fluorescence polarization of a fluorescently labeled protein upon transfer of glutamate from said labeled protein to a non-labeled protein by said transglutaminase.
detection of xyloglucan endotransglycosylase activity comprising measuring changes in fluorescence polarization of a fluorescently labeled oligo xyloglucan upon transfer of said labeled oligo xyloglucan to a non-labeled xyloglucan by said xyloglucan endotransglycosylase.
detection of pectin methyl esterase activity comprising measuring changes in fluorescence polarization of a fluorescently labeled methylated pectin upon demethylation by said pectin methyl esterase.
detection of arabinanase, activity comprising measuring changes in fluorescence polarization of a fluorescently labeled arabinan upon hydrolysis by said arabinanase.
detection of mannanase activity comprising measuring changes in fluorescence polarization of a fluorescently labeled galactomannan upon hydrolysis by said mannanase.
detection of rhamnogalacturonase activity comprising measuring changes in fluorescence polarization of a fluorescently labeled rhamnogalacturonan upon hydrolysis by said rhamnogalacturonase.
detection of cellulase activity comprising measuring changes in fluorescence polarization of a fluorescently labeled cellulose or cellulose derivative upon hydrolysis by said cellulase.

Still further the invention also encompasses new fluorescent substances which is particularly useful when applied in the screening method of the invention. Accordingly aspects of the invention concerns:
a fluorophore labeled cellulose and a process for producing this fluorescent substance.
a fluorophore labeled amylose and a process for producing this fluorescent substance.
a fluorophore labeled amylopectin and a process for producing this fluorescent substance.
a fluorophore labeled limit dextrin and a process for producing this fluorescent substance.
a fluorophore labeled galacaturonan and a process for producing this fluorescent substance.
a fluorophore labeled mannan and a process for producing this fluorescent substance.

### BRIEF DESCRIPTION OF THE DRAWINGS

### Figure 1

Schematic presentation of the steps of the screening procedure (1) = vial of host cell culture comprising a gene library; (2) = pre-propagation of transformants; (3) = dilution of transformants and/clones; (4) = separation and propagation of transformants or clones into microtiter wells of master plates; (5) = transfer of clones to assay plates and fluorescence polarization analysis of released biological compounds.

### Figure 2

A graphical presentation of Table 2.

### Figure 3

Results relating to Example 3 showing the decrease in fluorescence polarization over time upon hydrolysis of a fluorescein labeled starch by an amylase (squares) compared to a sample with no amylase present (triangles).

### Figure 4

Results relating to Example 4 showing a decrease in fluorescence polarization upon hydrolysis of a rhodamine labeled xylan by an xylanase (B) compared to a sample with no xylanase present (A).

### Figure 5

Results relating to Example 5 showing the increase in fluorescence polarization over time upon transfer of a rhodamine labeled cadaverine to a caseine by a transglutaminase (1) compared to a sample with no transglutaminase present (2).

### Figure 6

Results relating to Example 7 showing an increase in fluorescence polarization over time upon demethylation of a fluorescein labeled pectin by a pectin methyl esterase (PME) and subsequent cross-linking (1) with PME and 2 mM CaCl₂ added, (2) with PME but with no CaCl₂ added, (3) with 2 mM CaCl₂ but no PME added and (4) with neither CaCl₂ nor PME added.

### Figure 7

Results relating to Example 9 showing the decrease in fluorescence polarization upon hydrolysis of a fluorescein labeled pectin by a pectinase expressed by a transformants host cell (squares) compared to a sample with host cells expressing no pectinase activity (triangles).

### DETAILED DESCRIPTION OF THE INVENTION

### Definitions

As stated, *vide supra,* the term fluorescence polarization is in the present context abbreviated FP.

According to the principles of FP, if a fluorescent substance is excited by a beam of plane polarized light, it will in turn emit plane polarized light. The degree of polarization or the angle of the plane of the emitted light is inversely related to how much the fluorescent molecule has moved, e.g. rotated, between the time of excitation and emission. The time it takes for a molecule to rotate through an angle of approximately 68.5 degrees has been defined as the rotational relaxation time of the molecule. The rotational relaxation time is relatively small (about 1 nanosecond) for small molecules, and large (about 100 nanoseconds) for large molecules. The rotational relaxation time of a molecule is primarily dependent on its volume, so that the observed polarization of its fluorescence in solution gives a direct indication of its size.

In context of the invention, the term "nucleotide source" is to be understood as any DNA, RNA or cDNA material or material comprising DNA, RNA or cDNA.

In the context of the invention, the term expression system is to be understood as a system enabling transcription of a nucleotide sequence and translation into the synthesis of the corresponding biological compound. The expression system may be a cell or an *in vitro* system.

In the context of the invention, the term gene library is to be understood as fragments of DNA or cDNA derived from a nucleotide source.

In the context of the invention, the term "host cell" is to be understood as a cell, which may host and may express an inserted DNA or cDNA fragment from a gene library.

In the context of the invention, the term "transformant" or "transformed host cell" is to be understood as a host cell in which a DNA or a cDNA fragment from a gene library has been inserted.

In the context of the invention, the term "clone" is to be understood as a copy of a cell or a transformed host cell.

### The nucleotide source

In a preferred embodiment of the invention the nucleotide source is a cell, e.g. a prokaryotic cell, an archaeal cell or an eucaryotic cell. The cell may further have been modified by genetic engineering. A preferred bacterial cell is of the genus Bacillus, e.g. *B. licheniformis,* while a preferred eucaryotic cell is a mammal cell, e.g. a human cell, a plant cell, e.g. *Arabidopsis thaliana* or a fungus, e.g. *Meribipilus gigantus.*

In another preferred embodiment the nucleotide source is a mixed population of cells. The DNA or RNA of the cells may further be extracted, as described *vide infra,* directly from any biotic or abiotic sample, e.g. a soil sample, a water sample, or a rumen sample. Also preferred nucleotide sources are cells of extremeophile prokaryotics, such as thermophiles.

The nucleotide source may also be cells which have been subjected to classical mutagenesis, e.g. by UV irradiation of the cells or treatment of cells with chemical mutagens as described by Gerhardt et al. (1994).

Further the nucleotide source may be a population of cells genetically modified by *in vivo* gene shuffling as described in WO 97/07205.

In a further preferred embodiment the nucleotide source is in *vitro made* preparations of sequences of DNA, RNA, cDNA or artificial genes obtainable by e.g. gene shuffling (e.g. described by Stemmer, (1994) or WO 95/17413), random mutagenesis (e.g. described by Eisenstadt E. et al., (1994) or constructed by use of PCR techniques (e.g. described by Poulsen et al. (1991).

### The expression system

As previously defined, the expression system is a system enabling transcription of a nucleotide sequence and translation into the synthesis of the corresponding biological compound. The expression system may be cellular or an *in vitro* system. A description of *in vitro* coupled transcription and translation may be found in Ohuchi et al. (1998) or Ellman et al. (1991) enabling expression of a nucleotide sequence, e.g. a gene library derived from a nucleotide source. In the case of a cellular expression system, the cell may be the nucleotide source itself, e.g. a wild type cell or it may be a cell from a population of transformed host cells or clones thereof comprising a gene library prepared from a nucleotide source according to methods known to the art (e.g. described *vide infra).*

### The host cell

The host cell according to the definition may be any cell able of hosting and expressing a nucleotide fragment from a gene library.

A preferred host cell does not in itself contain or express nucleotide sequences encoding for biological compounds (i.e. untransformed host cells are unable of significantly expressing the biological compound), which will interfere with the FP screening method. This cell characteristic may either be a natural feature of the cell or it may be obtained by deletion of such sequences as described e.g. in Christiansen et al. (1997) or Stoss et al. (1997).

In another preferred embodiment of the invention the host cell is a bacterial cell or an eucaryotic cell. Further the bacterial cell is preferably a ElectroMAX DH10B (GibcoBRL/Life technologies, UK)cell or of the genus *E. coli,* e.g. SJ2 *E. coli* of Diderichsen et al. (1990). Other preferred host cells may be strains of Bacillus, such as Bacillus subtilis or Bacillus sp. A preferred eucaryotic cell is preferably a yeast, e.g. *S. cerevisae.*

### Preparation of the gene library

Preparation of a gene library can be achieved by use of known methods.

Procedures for extracting DNA from a cellular nucleotide source and preparing a gene library are described in e.g. Pitcher et al. (1989), Dretzen, G. et al. (1981), WO 94/19454,3969, Diderichsen et al. (1990)

Procedures for preparing a gene library from an *in vitro* made synthetic nucleotide source can be found in (e.g. described by Stemmer, (1994) or WO 95/17413).

### Insertion of a gene library into the host cell

Procedures for transformation of a host cell by insertion of a plasmid comprising a DNA or cDNA fragment from a gene library is well known to the art, e.g. Sambrook et al, 1989, Ausubel et al., 1995 and Harwood and Cutting, 1990.

In a preferred embodiment of the invention the plasmid to be inserted into a host cell also contains a nucleotide sequence (denoted as an antibiotic marker), which may enable resistance of a transformant to an antibacterial or antifungal agent e.g. an antibiotic. Resistance to chloramphenicol, tetracycline, kanamycin, ampicillin, erythromycin or zeocin is preferred.

In a further preferred embodiment of the invention the pSJ1678 plasmid DNA of WO 94/19454,3969 and Diderichsen et al. (1990), which enables resistance to chloramphenicol, may be used for transforming a SJ2 *E. coli* host cell. Alternatively the plasmid pZErO-2 (Invitrogen, CA, USA) may be used).

### Screening procedures

As stated *vide supra* the invention provides a method for screening for a nucleotide sequence encoding for a biological compound comprising:
a) expressing the nucleotide sequence in an expression system, so as to produce the biological compound,
b) contacting the biological compound with a fluorescent substance capable of reacting with the biological compound so as to let the reaction take place,
c) measuring the fluorescence polarization of the fluorescent substance, and
d) selecting cells or expression systems for which a change in fluorescence polarization has occurred.

In the case of an *in vitro* expression system (as described *vide supra)* as a specific embodiment of the invention the screening procedure preferably comprises:
a) preparing a gene library from a nucleotide source,
b) separating the gene fragments of the library into separate containers.
c) amplifying the separated gene fragments,
d) performing *in vitro* coupled transcription/translation of the amplified gene fragments so as to express a biological compound.
e) contacting the biological compound in each separate container or subsamples thereof with a fluorescent substance,
f) incubating biological compound with the fluorescent substance,
g) detecting FP changes of the fluorescent substance upon reaction with the biological compound.

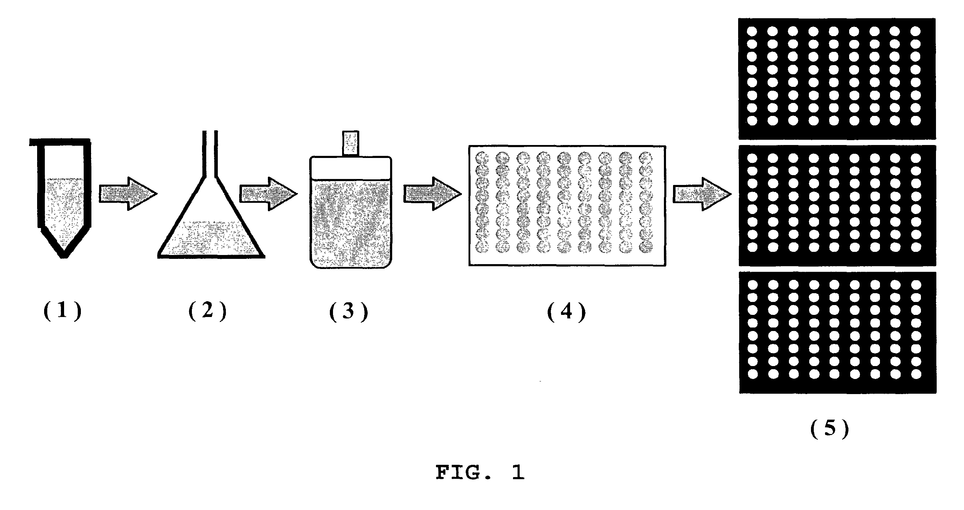

Steps a-g which may be schematically depicted as in Figure 1 ((1)+(2)+(3)=preparation of gene library; (4)=separation, amplification and transcription/translation; (5)=incubation with fluorescent substance and FP measurement) may suitably be achieved by use of commercially available standard equipment such as pipettes or automated pipette equipment, flasks, microtiter plates, shakers, thermostated incubators etc.

Changes in the fluorescent substance size upon reaction with a biological compound will create a change in FP, which can be detected by commercial FP equipment. Thus a change in FP will apply only for containers containing and expressing a gene fragment or nucleotide sequence encoding for a biological compound which reacts with the fluorescent substance(s) (from hereon and forward denoted as "positive hits").

The separation of gene fragments of the library may preferably be achieved by diluting the library to a degree which enables sampling of aliquots containing a gene fragment, preferably an average of one gene fragment per sample and then transferring the samples to separate containers, e.g. microtiter wells. The amplification of the separated gene fragments may be achieved by conventional PCR techniques as well as the *in vitro* coupled transcription/translation of the amplified gene fragments (see Ohuchi et al. (1998) page 4340 or Ellman et al. (1991) which is hereby incorporated by reference. Further preferred conditions for performing steps e), f) and g) is described below under cellular expression systems.

In the case of a cellular expression system (as described *vide supra)* as a specific embodiment of the invention the screening procedure preferably comprises:
a) pre-propagating and dilution of cells comprising the nucleotide sequence,
b) separating the cells into separate containers,
c) propagating separated cells to increase the number of clones of each cell in each separate container,
d) contacting the cells in each separate container with a fluorescent substance,
e) incubating cells with the fluorescent substance,
f) detection of FP changes of the fluorescent substance upon reaction with a biological compound released from the cells.

Steps a-f which may be schematically depicted as in Figure 1 ((1) +(2)+(3)=pre-propagation and dilution; (4)=separation; (5)=incubation with fluorescent substance and FP measurement) may suitably be achieved by use of commercially available standard equipment such as pipettes or automated pipette equipment, flasks, microtiter plates, shakers, thermostated incubators etc.

Pre-propagation and dilution of the cells may in one embodiment of the invention be designed to obtain a concentration of cells per volume suitable for sampling aliquots containing an optimal number of cells to be separated. A suitable average number cells per aliquot may be 0,3-10, preferably 0,3-5, e.g. 0,3-1.

Pre-propagation (picture 1 in figure 1) of the cells in a preferably aqueous medium preferably provides a 2-5 times increase in the number of cells clones. Suitable incubation temperatures may be within the range of 10-60°C, preferable 30-50°C, e.g. 37 °C, while pH may be kept between 4-10, preferably 6-8. The incubation period should be adjusted, preferably 15-60 minutes, e.g. 40 minutes, so as to meet the requirements of the desired transformant and clone concentration.

In the case of the expression system being a culture of host cells wherein transformants comprises a gene library derived from a nucleotide source, pre-propagation may also be performed to secure expression of an antibiotic marker which may be comprised in the inserted plasmid of transformed host cells enabling resistance to an antibiotic in the medium. Pre-propagation of the host culture may accordingly be achieved by incubation at conditions favorable for expression of the chosen type of antibiotic marker as well as securing viability of the transformant.

Dilution (picture 2 of figure 1) may be performed by addition of a medium, to ensure that all cells and clones thereof resides in the diluted solution.

The cells may be separated by transferring aliquots of the planned specific volume to separate containers, e.g. wells in commercial microtiter plates (picture 3 of figure 1).

Alternatively the separation may be performed including a prescreening by use of a FACS machine as described in WO 98/58085, which is hereby incorporated by reference. Accordingly step b) may comprise the steps:
1) inserting a fluorescent substance into the cells characterized by changing emission intensity change upon reaction with a biological compound inside the cells,
2) screening, selecting and separating cells for which a change in emission intensity has occurred by means of a FACS machine.

The separated cells are propagated to increase the number of clones in each container, preferably to a range between 10⁷-10⁸ clones/ml. If microtiter plates are used these plates may be denoted "master plates". For screening a bacterial gene library 50-100 master plates with each 96 wells may typically be employed. One advantage of having a master plate is the possibility of keeping viable samples of the cells to be screened, so that even if the subsequent screening conditions results in death of the screened cells, it is possible to track back a screening result to a viable sample of a screened cell.

In a further preferred embodiment suitable incubation temperatures may be in the range of 10-60°C, preferable 30-50°C, e.g. 37°C, while the pH may be kept between 4-10, preferably 6-8. The incubation medium should meet the nutritional requirements of the cells and clones to ensure sufficient growth.

Also in the case of the expression system being a culture of host cells wherein transformants comprises a gene library derived from a nucleotide source and an antibiotic marker a medium may suitable be chosen enabling killing or suppressing non-transformed host cells.

Incubation times should be adjusted so as to ensure growth yielding a sufficient number of cells/clones suitable for sampling aliquots containing a suitable number of clones for screening leaving a number of viable clones in the master plate. A preferred propagation period may be 40-90 hours, e.g. 48 hours, depending on the type of microbial host cell and the propagation conditions.

In the case of the expression system being a culture of host cells comprising a gene library in which an antibiotic marker is comprised in transformed host cells the pre-propagation, dilution and/or propagation, in a preferred embodiment of the invention, are performed in a medium capable of selectively killing or suppressing growth of non-transformed host cells. This may preferably be achieved by adding e.g. an antibiotic to the culture medium towards which the transformants or clones thereof are resistant, in an amount effective on non-transformed host cells.

Aliquots of the cells are transferred, e.g. by pipette, to a microtiter assay plate (picture 4 in figure 1) suitable for measuring FP e.g. black microtiter plates from Bibby Sterilin Ltd., UK or Dynex Technologies, VA, USA. The cells/clones are contacted with one or more fluorescent substances having FP properties, e.g. by adding a substance mixture to each well. The fluorescent substance(s) should be suitable for reaction with the type of biological compound(s) of interest (e.g. fluorescently labeled pectin which is suitable for reaction with pectinase enzymes released from a cell). The contact between the biological compound and the fluorescent substance(s) usually occur in the extracellular medium, a condition which may be required in order to obtain an adequate FP response. In case the biological compound is confined within the interior of the cell, the cell may lysed or its integrity otherwise disrupted in order to release the biological compound to the medium.

The mixture of cells, medium and fluorescent substance(s) are incubated so that a biological compound may react with the fluorescent substance(s).

Changes in the fluorescent substance size upon reaction with a biological compound will create a change in FP, which can be detected by commercial FP equipment. Thus a change in FP will apply only for cells containing and expressing a nucleotide sequence encoding for a biological compound which reacts with the fluorescent substance(s) (from hereon and forward denoted as "positive hits").

The incubation may be performed at conditions, which favor the reaction between the biological compound and the fluorescent substance(s). In a preferred embodiment the reaction between the fluorescent substance(s) and the biological compound is optimized with respect to pH and temperature. The incubation may also be performed at extreme conditions (such as very low temperatures (e.g. below 30°C or below 20°C) or high temperatures (e.g. above 60°C or above 70°C), low pH (e.g. below 5 or below 4) or high pH (e.g. above 9 or above 10), low or high ionic strength, presence of hostile chemicals such as detergents) causing death of the cells, depending on which biological compound is to be detected. If for instance the biological compound to be found is a thermostabile compound the incubation may be performed at high temperatures, conditions providing that only biological compounds, which remain active at high temperatures will react with the fluorescent substance.

Positive hits are preferably identified as cells/clones or *in vitro* expression systems generating a FP change, which is more than 3.5 standard deviations of the based on the variance in background FP of the chosen fluorescent substance at the chosen conditions. As the content of each well in a microtiter plate is very homogeneous the variation from well to well becomes little despite the presence of cloning host cells (see the examples 27 and 28). The standard derivation within each 96 wells plate is typically 2 to 3% when a soluble fluorescently labelled substrate (e.g. soluble starch) is being applied. However, some substrates which do not dissolve that well (e.g. fluorescently labelled amylose and PASC) give rise to a higher variation typically 5 to 7% standard derivation. Due to the high accuracy by which gene libraries can be screened by the use of fluorescence polarisation the assays become very sensitive making it possible to detect enzymatic activities even though the expression level is low or the specific activity is low under the applied assay conditions.

DNA in a cellular nucleotide source or a gene library derived from a nucleotide source and expressed either in an *in vitro* expression system or by transformation into a host cell expression system may thus be screened for nucleotide sequences encoding for biological compounds which reacts with a chosen range of fluorescent substance(s).

In one preferred embodiment of the invention the reaction between the fluorescent substance and a biological compound is measured in real time by placing the assay plate in a FP equipment and measuring changes in FP during incubation.

In another preferred embodiment of the invention a reaction between the fluorescent substance and a biological compound is detected by measuring changes in FP after a predefined time of incubation compared to a background measurement of the fluorescent substance alone (endpoint measurement).

Techniques for measuring FP and the description of modern equipment is available with commercial equipment, e.g. Jolley Consulting and Research, Inc, IL, USA). Other reference can be found in Checovich et al. (1995).

When the positive hits has been identified it may be verified, that the found biological compound originates from clones of a single cell by employing conventional screening techniques or by repeating the method of the invention. This may be achieved by restreaking the clones onto a suitable agar plate and assaying the thereby obtained single colonies for the biological compound in question.

### The biological compound

The biological compound in the context of the invention may be any compound subject to industrial applicability which may be expressed in an expression system.

In a preferred embodiment of the invention the biological compound may be proteins, polypeptides or peptides

A preferred biological compound is an enzyme, which in accordance with *Recommendations (1992) of the Nomenclature Committee of the International Union of Biochemistry and Molecular Biology,* Academic Press, Inc., 1992 may be classified as e.g. an oxidoreductase (EC 1.-.-.-), a transferase(EC 2.-.-.), a hydrolase (EC 3.-.-.-), a lyase (EC 4.-.-.-), an isomerase (EC 5.-.-.-), a ligase (EC 6.-.-.-).

Said hydrolase may be an endo-, exo-, carboxy- or amino peptidase, an endo- or exo glucanase, an endo- or exo amylase, a lipase or an esterase.

The biological compound may further have a medical indication on a human or animal body, e.g. a hormone or other proteins, polypeptides or peptides, which acts on human or animal receptors.

Further a preferred biological compound is an extra cellular compound such as an extra cellular enzyme, i.e. a compound which is secreted or otherwise exported from a cell to its surrounding medium. In some cases the biological compound being an extra cellular compound is a necessity because the compound may not be active when confined within a cell. For example some enzymes such as certain proteases are synthesized as inactive precursors, e.g. having attached a signal peptide or propeptide rendering the enzyme inactive as long as it is inside the cell. Upon export from the cell the signal peptide may be cleaved off rendering the enzyme active, when entering the surrounding extra cellular medium (Simonen and Palva (1993)). Some pullulanases and lipases do also show lack of activity or reduced activity in the cytoplasm and are activated upon secretion (Gotz et al. (1998) and Pugsley et al. (1990).

### The fluorescent substance

The substance in the context of the invention may be any organic or biological compound which in itself posses fluorescence properties or which is labeled with a fluorescent marker. In the context of the invention the skilled person may choose from a broad range of possible fluorescent substances, because the method is based on measurement of FP on samples of constant fluorescence intensity, i.e. changes in the polarization of emitted light of a constant intensity. This has several advantages as compared to methods based on measurement of changes in intensity of a fluorescent substance:
i) No reaction between the biological compound and the fluorogenic part (fluorophore) of the fluorescent substance is necessary as there is no need to change the properties of the fluorophore to change the emission intensity. Accordingly in the context of the invention useful fluorescent substances are not limited to substances designed to achieve a change in intensity upon reaction with the biological compound (such fluorescent substances often comprises a fluorophore and a quenching component which must be sterically situated at a certain distance to each other.
ii) This in turn makes it possible to design suitable fluorescent substances, e.g. by labeling techniques, which also in turn enables the making of fluorescent substances, which have a high resemblance to substances with which the biological compound will react in an intended real life industrial application. Accordingly there is no limitation to fluorescent substances designed to e.g. penetrate cells and stay confined within the screening procedure.
iii) High resemblance between the fluorescent substance and substances with which the biological compound will react in an intended real life industrial application renders the screening method specific and highly versatile, i.e. applicable for many kinds of different biological compounds.
iv) The principle in FP is a ratio measurement providing a better signal to noise ratio than for methods based on measurement of emission intensity, i.e. less background noise disturbs the measurement.
v) FP assays are true homogeneous assays, which means that no wash or separation of e.g. unreacted fluorescent substance is needed.

In a preferred embodiment the fluorescent substance is an organic molecule labeled with a fluorescent marker (fluorophore). The organic molecule may be a poly-, oligo- or monomer and may further be labeled with more than one fluorescent markers. Markers on the organic molecule may be attached at 0,01-10% of the available bonding sites of the organic molecule (degree of labeling). A preferred range is 0,5-5%, e.g. 2 %. The fluorescent marker may be attached to the organic molecule by covalent, ionic or hydrogen bonding.

The organic molecules may preferably be proteins, peptides, glycoproteins, lipoprotein, mono-, poly- or oligosaccharides, triglycerides or nucleotides. Further the organic molecule may act as a substrate or an inhibitor, a human or animal receptor, a mono- or polyclonal antibody for the biological compound encoded for by the nucleotide sequence for which the cell or the gene library is to be screened for. Preferably the organic molecule is soluble or dispersible in an aqueous medium.

Preferred proteins may be casein, derivatives of casein, gelatins, albumins, soy protein, polyclonal antibodies, monoclonal antibodies, human receptors or an animal receptors, e.g. tissue factor as described in national patent DK/97/0879 or WO 99/03498. A preferred peptide is cadaverine.

Preferred polysaccharides may be glucans such as amyloglucans (e.g. amylose and/or amylopectin), dextrins (e.g. limit dextrin), pectins/polygalacturonic acids, celluloses (e.g. phosphoric acid swollen cellulose as described in Schülein (1997)) or derivatives thereof or hemi-celluloses. Further preferred hemicellulose may be xyloglucans, xylans, arabinoglucans or arabinans. Also mannans (such as galactomanan) and galacturonan (such as rhamnogalacturonan) are useful.

Other useful organic molecules are phenolic molecules such as phenolic polysaccharides which by oxidation by an oxidoreductase may be polymerized.

The organic molecule may be chemically or enzymatically synthesized. Alternatively it may be obtainable from an animal, human, plant or microbial source and may further subsequently be chemically or enzymatically modified and/or purified.

The marker may suitably have an excitation maximum in the range 350 - 700 nm, e.g. 400 - 600 nm, while the emission maximum suitable may be in the range 450-800 nm, e.g. 500-700 nm.

The marker may preferably be less liable to be quenched by conjugating or attaching it to the organic molecule so that it maintains a distance of 20-50 Ångstrom from the organic molecule and other fluorescent markers. Also the bond between the marker and the organic molecule preferably is a rigid bond limiting rotational movement of the marker itself around the bond axis, independently of the movement of the organic molecule, so that the movement of the marker is primarily or essentially governed by the rotational movement of the organic molecule (around the rotation axis of the organic molecule).

The marker may further have a fluorescence relaxation time in the range of 1-7 nanoseconds, preferably 3-5 nanoseconds.

Preferred markers are derivatives of BODIPY, fluorescein including derivatives thereof, rhodamine including derivatives thereof, dansyls including derivatives thereof (e.g. dansyl cadaverine), Texas red (all available from Molecular Probes, OR, USA) as well as cyanine dyes (available from Amersham, Buckinghamshire, UK)

The fluorescent substance (organic molecule and fluorescent marker(s) in combination) should have a polarization value in the range of 50-500 mP (milli polarization units), preferably 100-300 mP, e.g. 200 mP.

The invention also encompasses new fluorescent substances which is particularly useful when applied in the screening method described *supra.* One preferred fluorescent substances is a fluorophore labeled cellulose. For example phosphoric acid swollen cellulose (PASC) or carboxy methyl cellulose (CMC) may be labeled with a fluorophore such as 6-DTAF (6-Fluorescein dichlorotriazine). A process for producing a fluorescent cellulose may comprise: i) suspension or dissolution of the cellulose or derivative thereof in an aqueous alkaline reaction medium; ii) incubation/reaction of the suspended or dissolved cellulose or derivative thereof with the fluorophore; iii); separation of the labeled cellulose or derivative thereof from the alkaline reaction medium and wash of the labeled cellulose and iv) neutralization of the labeled cellulose or derivative thereof.

Another preferred fluorescent substance is a fluorophore labeled galacturonan. For example rhamnogalacturonan may be labeled with a fluorophore such as 6-DTAF (6-Fluorescein dichlorotriazine). A process for producing a fluorescent rhamnogalacturonan may comprise: i) Suspension and/or dissolution of the rhamnogalacturonan in an alkaline aqueous medium ii) incubation/reaction of the suspended or dissolved rhamnogalacturonan with the fluorophore; iii) neutralization of the alkaline reaction medium, iv) separation of the labeled rhamnogalacturonan from the neutralized reaction medium and wash of the labeled rhamnogalacturonan.

Yet, another preferred fluorescent substance is a fluorophore labeled mannan. For example galactomannan may be labeled with a fluorophore such as 6-DTAF (6-Fluorescein dichlorotriazine). A process for producing a fluorescent galactomannan may comprise: i) Suspension and/or dissolution of the galactomannan in an alkaline aqueous medium ii) incubation/reaction of the suspended or dissolved galactomannan with the fluorophore; iii) separation of the labeled galactomannan from the alkaline reaction medium and wash of the labeled galactomannan.

Yet, another preferred fluorescent substance is a fluorophore labeled xylan. For example xylan may be labeled with a fluorophore such as lissamine-rhodamine- sulphonyl chloride. A process for producing a fluorescent xylan may comprise: i) Suspension and/or dissolution of the xylan in an alkaline aqueous medium ii) amidation of the xylan; iii) incubation/reaction of the amidated xylan with the fluorophore; iv) separation and wash/purification of the labeled xylan.

Yet, another preferred fluorescent substance is a fluorophore labeled xyloglucan. For example xyloglucan may be labeled with a fluorophore such as fluorescein-5-thiosemicarbazide. A process for producing a fluorescent xyloglucan may comprise: i) suspension and/or dissolution of the xyloglucan in an aqueous medium comprising an oxidant ii) separation/purification of a partially oxidized xyloglucan fraction of interest iii) incubation/reaction of the oxidized xyloglucan with the fluorophore; iv) separation and wash/purification of the labeled xyloglucan.

Yet, another preferred fluorescent substance is a fluorophore labeled arabinan. For example arabinan may be labeled with a fluorophore such as fluorescein-5-thiosemicarbazide. A process for producing a fluorescent arabinan may comprise: i) suspension and/or dissolution of the arabinan in an aqueous medium comprising an oxidant ii) separation/purification of a partially oxidized arabinan fraction of interest iii) incubation/reaction of the oxidized arabinan with the fluorophore; iv) separation and wash/purification of the labeled arabinan.

Yet, another preferred fluorescent substance is a fluorophore labeled pectin/polygalacturonic acid. For example pectin may be labeled on carboxylic acid groups with a fluorophore such as fluorescein-5-thiosemicarbazide using a mediator such as 1-ethyl-3-(dimethylaminopropyl)carbodiimide hydrochloride. A process for producing a fluorescent pectin may comprise: i) suspension and/or dissolution of the pectin in an aqueous medium ii) incubation/reaction of the pectin with the fluorophore and the mediator; iv) separation and wash/purification of the labeled pectin.

### Other uses

The FP methods described *vide supra* may further be used for quality or fermentation control in industrial production of biological compounds within the scope of the invention.

### MATERIALS AND METHODS

### Deposited microorganisms

The following microorganisms were deposited by the applicant according to the Budapest Treaty on the International Recognition of the Deposits of Microorganisms for the Purpose of Patent Procedures at Deutsche Sammlung von Mikroorganismen und Zellkulturen GmbH:
1999-02-03; Bacillus RCS 242B; DSM accession No. 12663
1999-02-03; Bacillus RCS 245B; DSM accession No. 12664
1999-02-15; B. subtilis MB208; DSM accession No. 12682
1999-02-26; Bacillus sp.; DSM accession No. 12708
1999-02-26; Bacillus sp.; DSM accession No. 12709

### Construction of gene libraries

The bacterial strain from which DNA was extracted for library construction was propagated in liquid LB medium. Cells were harvested, and genomic DNA isolated by the method described by Pitcher et al., 1989.

In examples 25 and 26, genomic DNA was partially digested with restriction enzyme Sau3A, and size-fractionated by electrophoresis on a 0.7 % agarose gel. Fragments between 2 and 7 kb in size were isolated by electrophoresis onto DEAE-cellulose paper (Dretzen, G., et al., 1981).

Isolated DNA fragments were ligated to BamHI digested pSJ1678 (see WO 94/19454,3969) plasmid DNA, and the ligation mixture was used to transform the strain *E. coli* SJ2 (Diderichsen et al., (1990)).

*E. coli* SJ2 cells were prepared for and transformed by electroporation using a Gene PulserTM electroporator from BIO-RAD as described by the supplier. All molecular biology work was done as described by Sambrook et al, 1989, Ausubel et al., 1995 and Harwood and Cutting, 1990.

In examples 27-29, libraries generated in pZErO-2 (Invitrogen, CA, USA) were created as follows: Chromosomal DNA was partially digested with restriction enzyme Sau3A and subsequently size fractionated by gel electrophoresis. Fragments of 2-8 kb were purified and ligated into pZErO-2 restricted with BamHI. The ligation mix was transformed into ElectroMAX DH10B (GibcoBRL/Life technologies, UK) cells by electroporation as described by the vendor. The transformed cells were grown in LB medium without antibiotics for 60 minutes, glycerol was added to 15% and frozen in aliquots. The number of transformants per aliquot was determined by plating on LB agar plates containing 10 µg/ml kanamycin.

### Handling of microtiter plates and propagation of E. coli SJ2 in microtiter plates.

For handling of the master plates a Titertek Microdrop station equipped with an S20 stacker (Life Technologies, MD, USA) was used for automated filling of the transformed library into the wells. For transfer of cells from master plates into assay plates a Platemate automated pipettor (Matrix Technologies, MA, USA) station was used.

SJ2 *E. coli* transformants and clones thereof were propagated in a LB broth medium (5g/l NaCl, 5g/l yeast extract (Difco, MI, USA), 10 g/l Tryptone (Difco, MI, USA)). The *E. coli* transformants and clones thereof were propagated in Nunclon MicroWell 96F plates (NUNC, Denmark) at 37°C and shaked at 200 rpm for three days prior to the screening procedures. Prior to FP measurements transformants or clones thereof and fluorescent substance are mixed by mixing 50 µL transformants or clones thereof with 150 µL solution comprising the fluorescent substance.

### Determination of fluorescence polarization.

For fluorescence polarization measurements in cuvettes an LS50B (Perkin Elmer, CT, USA) equipped for fast polarization was used and for measurements in microtiter plates an FPM-2 (Jolley Consulting and research, Inc., IL, USA) was applied. The variation of repeated measurements in the same well and same sample in several wells have showed that the standard variation is from 2% to 4%. Two types of black microtiter plates were used:
Sterilin flat bottom well plates (Bibby Sterilin Ltd., UK) and Microfluor U-bottom plates (Dynex Technologies, VA, USA).

For measuring fluorescein in microtiter plates an excitation filter was inserted allowing 485 nm light to pass with a band pass of 22 nm. Further an emission filter was inserted allowing 520 nm light to pass with a band pass of 30 nm.

For measuring rhodamine in microtiter plates an excitation filter was inserted allowing 546 nm light to pass with a band pass of 12 nm. Further an emission filter was inserted allowing 590 nm light to pass with a band pass of 35 nm.

The grating factor was calculated according to the instrument instruction manual.

Instruments for high through put measurements of fluorescence polarization may by a flow cuvette based instrument e.g. modified LS50B (Perkin-Elmer Corporation, CT, USA), microtiter plate based readers e.g. Polarstar (BMG, Germany) or a FACS (flow activated cell sorter) equipped for fluorescence polarization as described by Arndt D.J., et al. (1976) and Lindmo, T and H.B. Steen, 1977.

### Enzymes and substrates used in the assays.

Cadaverine labeled with dansyl, tetramethyl rhodamine, BODIPY® -TR and Texas Red were obtained from Molecular Probes, OR, USA. Dephosphorylated α-casein from bovine milk and fluorescein labeled potato starch were obtained from Sigma, MO, USA. Citrus pectin with a low degree of esterification (3%) was obtained from Copenhagen Pectin, Lille Skensved, Denmark. Apple pectin with a degree of esterification of 77,4% was from Herbstreith KG, Germany. Birch xylan used in the xylanase experiment was obtained from Sigma, MO, USA. Arabinan was from sugar beet and xyloglucan, (amyloid) from Tamaring seed were both obtained from Megazyme (Sydney, Australia). Amylose from potato (type III) and amylopectin from corn were both obtained from Sigma, MO, USA. Galactomannan from Locus bean gum was obtained from Sigma, MO, USA. Rhamnogalacturonan was obtained from Copenhagen Pectin, Lille Skensved, Denmark. Phosphoric acid swollen cellulose was prepared as described in Schülein (1997). Limit dextrin was obtained by hydrolysing corn-starch by an amylase from *Aspergillus oryzae* as described Tada et al. (1989).

Polygalacturonases I, II, III of WO 9414952 were used. A pectin lyase obtainable from *Aspergillus aculeatus* as described in WO 94/21786 was used. A pectate lyase obtainable from *Bacillus licheniformis* as described in national patent application number DK/97/1344 was used. An arabinanase obtainable from *Aspergillus* *aculeatus* as described in WO 94/20611 was used. A xyloglucanase (endoglucanase II) obtainable from A. aculeatus as described in WO 94/14953 was used. An amylase (Termamyl® ) available from Novo Nordisk A/S, Denmark was used. A pectin methyl esterase obtainable from A. aculeatus as described in Christgau et al., 1996 was used. A transglutaminase obtainable from *Phytophthora cactorum* as described in WO 96/23366 was used. A partially purified potato xyloglucan endotransglycosylase purified from potato as described by Fry et al., 1992 was used. A xylanase (BioFeed® Wheat) available from Novo Nordisk A/S Denmark was used. A mannanase obtainable from A. aculeatus as described in WO 94/25576 was used. The amylase Novamyl is available from Novo Nordisk A/S and the β-amylase from *C. thermosulfurogenes* was obtained as described in Shen et al. (1988). A rhamnogalacturonase obtainable from *A. aculeatus* as described in WO 94/20612 was used. A Cel45 cellulase obtainable from *Humicola insolens* as described in WO 96/29397 was used.

### BP-X medium

BP-X medium was prepared as follows: 100 g potato flour, 50 g barley flakes and 800 mg BAN amylase (available from Novo Nordisk A/S, Denmark) was mixed in 800 ml water. The solution was heated to approximately 70°C and incubated for 30 minutes. During cooling 10 g sodium caseinat (MD-Food , Denmark) was added. Upon cooling to ambient temperature 20 g soy meal, 9 g di-sodiumhydrogenphosphate (MERCK), 0.1 g pluronic PE 6100 (BASF) was added along with additional 1000 ml water.

### TY medium

TY medium was prepared as described in Ausubel et al. (1995)

### EXAMPLES

The invention is illustrated by the following non limiting examples:

### Example 1, labeling of oligo-xyloglucan

Oligo-xyloglucan was end-labeled with sulphorhodamine as described by Fry et al.1997.

### Example 2; labeling of xyloglucan by partial chemical oxidation

Xyloglucan (0.50 g) was dissolved in water (60 ml) at 60°C by stirring for 7 h. The solution was allowed to cool to room temperature and a solution containing sodium metaperiodate (15 mg) (Merck, Germany) in water (10 ml) was added. The mixture was left overnight in the dark and then dialyzed (MW cut-off 12.000-14.000) against demineralized water with frequent exchange of the acceptor solution. 25 mg fluorescein-5-thiosemicarbazide supplied by Molecular Probes, OR, USA) was dissolved in 1 ml DMF (Sigma, MO, USA) and the now orange solution was added to the dialyzed polymer (~ 60 ml solution). The solution was stirred in the dark for 18 h and the polymer then precipitated by addition of 96 % ethanol (20 ml). The wet polymer was collected by filtration on Miracloth (Calbiochem, Germany) and washed with a mixture of water and ethanol (1:1) until a colorless filtrate was obtained. The labeled yellow xyloglucan was finally washed with absolute ethanol and air dried. The estimated degree of labeling (calculated from absorbance at 492 nm) was 0.6%.

### Example 3; labeling of arabinan by partial chemical oxidation

Arabinan (0.50 g) was dissolved in water (10 ml) and a solution containing sodium metaperiodate (15 mg) in water (5 ml) was added. The mixture was left overnight in the dark and then diluted with water (15 ml). The solution was dialyzed (MW cut-off 12.000-14.000) against water with frequent exchange of the acceptor solution. 25 mg fluorescein-5-thiosemicarbazide supplied from Molecular Probes, OR, USA was dissolved in 1 ml DMF and the now orange solution was added to the dialyzed polymer. The solution was stirred in the dark for 36 h and the solution then split into two fractions. One fraction was concentrated to a smaller volume and the polymer precipitated from 90% ethanol. Estimated degree of labeling (calculated from absorbance at 492 nm) was 0.7%.

### Example 4; labeling of polygalacturonic acid

The labeling strategy comprised labeling of the carboxylic acid groups of pectin/polygalacturonic acids by coupling a hydrazine or amine activated probe (e.g. fluorescein-5-thiosemicarbazide) to the carboxylic acids using 1-ethyl-3-(dimethylaminopropyl)carbodiimide hydrochloride (FLUKA, Switzerland) as a mediator.

The polygalacturonic acid with a degree of esterification of 3%, 35% or 77% (1 g) was dissolved in 25 ml of demineralized water (additional heating and water was needed to dissolve pectin with degrees of esterification of 35% and 77%) and subsequently 20 mg of fluorescein-5-thiosemicarbazide (Molecular Probes, OR, USA) was applied while stirring. The pH was adjusted to 5.9 with 1 M NaOH and (1 g) 1-ethyl-3-(dimethylaminopropyl)carbodiimide hydrochloride was added gradually over 1 h while stirring. The solution was then incubated overnight in darkness. 10% acetic acid (v/v) was added to the solution followed by addition of methanol to a final concentration of 75% (v/v) methanol. This solution was placed in darkness for 1 h. The precipitated pectin was subsequently collected by filtration on 2 layers of Miracloth (Calbiochem, Germany) and washed with acetic acid:ethanol (1:19 v/v), ethanol (96%) and finally with acetone. The labeled pectin was dried in darkness. The degree of labeling were between 0.3 and 0.7 %.

### Example 5; labeling of xylan

2.0 g of xylan was suspended in 50 ml water at 90°C and subsequently cooled to 30 °C. 17.5 g solid ethylene diamine dihydrochloride (FLUKA, Switzerland) was dissolved and pH adjusted by addition of 10 M NaOH to pH 5.9 at 25°C. 1-ethyl-3-(dimethylaminopropyl)carbodiimide hydrochloride (5 g) was slowly added and the mix was stirred overnight at 25°C. The solution was diluted with 125 ml water and subsequently 12.5 ml acetic acid and finally 375 ml methanol was added. The xylan precipitate was collected by filtration on 2 layers Miracloth (Calbiochem, Germany) in a 12-cm funnel. The moist xylan was re-dissolved in pyridine/acetic acid/H₂O (1:1:18 by vol.) to a final volume of 125 ml. 375 ml methanol was added to the mix and incubated for 1 hour. The precipitate of xylan was collected on Miracloth as before. The xylan was rinsed with 1 liter of methanol/pyridine/acetic acid/H₂O (60:1:1:18 by vol.) followed by 1 liter of ethanol and finally with 0.5 1 acetone. The amidated polysaccharide was collected and dried. 1.40 g of amidated xylan was dissolved in 35 ml hot 200 mM sodium pyrophosphate solution. After dissolution of the amidated xylan the solution was cooled to room temperature and pH adjusted to 9.5. 1% (w/v) lissamine-rhodamine- sulphonyl chloride (Molecular Probes, OR, USA) in dry dimethyl-formamide was slowly over 3 hours added to the xylan. The solution was dialyzed (cut off at 10.000-12.000) against 3 x 1 liter demineralized water containing 0.5% 1,1,1-trichloro-2-methylpropan-2-ol. The content was recovered and 2 ml acetic acid and 2 ml pyridine was added followed by 150 ml methanol to precipitate the xylan. The precipitated xylan was re-dissolved in 50 ml pyridine/acetic acid/H₂O (1:1:18 by vol.) and reprecipitated by addition of 150 ml methanol. The washing step was repeated until the supernatant did not contain any dye. Precipitated xylan was washed twice in 200 ml acetone and dried.

### Example 6; labeling of Phosphoric Acid Swollen Cellulose(PASC)

250 ml of an 10 % aqueous solution of PASC (≈ 2.5 g cellulose) was adjusted to pH 10.5 with 0.5 M NaOH and incubated overnight in the dark with 6-DTAF (6-Fluorescein dichlorotriazine). The reaction mixture was centrifuged and the supernatant removed. The labelled polymer was washed 5 times using 100 ml 0.1 M NaOH solution. Each wash was followed by centrifugation and removal of the supernatant. The washed labelled polymer was neutralised in 2 x 250 ml of water and resuspended in 250 ml water.

### Example 7; labeling of amylose

Amylose (250 mg) was refluxed for 10 min. at 100 °C in water (30 ml) and pH adjusted to pH 11 with NaOH. The turbid solution was cooled to room temperature and treated with 11 mg 6-DTAF (6-Fluorescein dichlorotriazine). The labeled polymer was precipitated in a mixture of EtOH/MeOH/AcOH (15:2:1, 20 ml) and collected on Miracloth and washed with EtOH (96 %, 30 ml) and acetone (140 ml). The polymer was finally dissolved in water and freeze-dried.

### Example 8; labeling of amylopectin

Amylopectin (250 mg) was refluxed for 10 min. at 100 °C in water (30 mL) and pH was adjusted to 11 with NaOH. The turbid solution was cooled to room temperature and treated with 11 mg 6-DTAF (6-Fluorescein dichlorotriazine). The labeled polymer was precipitated in a mixture of EtOH/MeOH/AcOH (15:2:1, 15 ml) and washed with EtOH (96 %, 30 ml). The precipitate was collected on Miracloth and washed with EtOH (96 %, 70 ml). The polymer was finally dissolved in water and freeze-dried.

### Example 9; Labeling of limit dextrins

Corn-starch was hydrolyzed by an amylase from *Aspergillus oryzae* (Tada et al. (1989) at pH 4.5, 50°C for 120 h. The limit dextrins were purified on Bio-gel P-2 column. Fractions containing DP 15-33 were pooled and freeze-dried. 508 mg dextrins were re-dissolved in 20 ml water and 75 mg 5-aminofluorescein in 1 ml DMF was added and stirred for 2 h in the dark. The resulting imine-bond was reduced using 76.2 mg NaCNBH₃. The labeled dextrins was purified on a SEC-column.

### Example 10; labeling of rhamnogalacturonan

12 mg 6-DTAF (6-Fluorescein dichlorotriazine) was added to an aqueous 1.5% solution of rhamnogalacturonan (Copenhagen Pectin, Lille Skensved, Denmark) (~225 mg in 15 ml) adjusted to pH 11 with 1 M NaOH. The solution was stirred overnight in the dark and neutralized with 0.5 M HCl. EtOH (30 ml) was added, the polymer collected on Miracloth and washed with EtOH/water (2:1, 150 ml) and then with EtOH (400 ml). The polymer was dissolved in water (25 ml) and freeze-dried.

### Example 11; labeling of galactomanan

2.695 g galactomannan was dissolved in 250 mL water adjusted to pH10 using 0.5 M NaOH. 0.127 g DTAF (6-Fluorescein dichlorotriazine) was added and the solution was stirred in the dark for 24 hours. The product was precipitated in 500 ml EtOH and was subsequently washed 3 times with 250 ml EtOH and once in 250 ml acetone. The solvent was removed by evaporation. The labeled polymer was re-dissolved in 400 ml water.

### Example 12; amylase activity assayed by fluorescence polarization

A method for detection of amylase activity was performed by incubating an amylase (Termamyl® ) with a fluorescein labeled potato starch according to example 7 and measuring the changes in fluorescence polarization. A decrease in fluorescence polarization of the fluorescent starch upon hydrolysis by the amylase was observed as shown in Table 1.

**Table 1**

| **Results** | + Termamyl® | - Termamyl® |
|---|---|---|
| FP value | 126 mP | 166 mP |

The hydrolysis reaction was conducted by incubating Termamyl® with 33 µg fluorescein labeled potato starch per well for 180 minutes at ambient temperature in a 200 µL volume of pH 6.8 BR-buffer (23 mM H₃PO₄, 23 mM CH₃COOH, 23 mM H₃BO₃). After the 180 min reaction time the decrease in fluorescence polarization was approximately 20%.

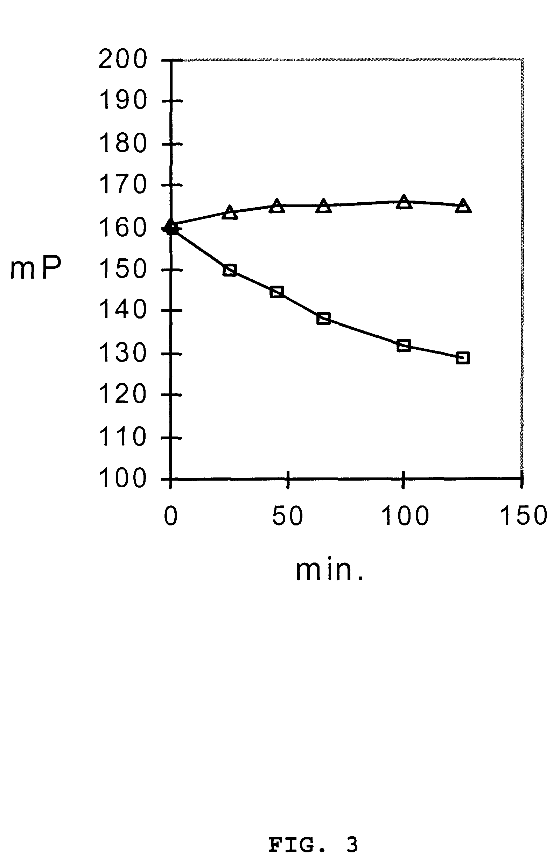

Figure 3 shows the FP changes over time, thus demonstrating the rate of which the starch is hydrolyzed by the amylase. FP measurements of the fluorescent starch with and without addition of Termamyl® are indicated by squares and triangles respectively.

An amylase expressed by a cloned *E. coli* SJ2 transformant could also be detected by fluorescence polarization. A 50 µl aliquot was taken from a master plate propagated overnight at 37°C with shaking. The aliquot was mixed in a microtiter well with 150 µl assay buffer pH 6.8 (23 mM H₃PO₄, 23 mM CH₃COOH, 23 mM H₃BO₃) containing 33 µg fluorescein trace labeled starch. After incubation for 3 hours at ambient temperature the decreases in FP were measured as shown in Table 2.

**Table 2**

| **Results** | + amylase | - amylase |
|---|---|---|
| FP value | 100 mP | 133 mP |

### Example 13; xylanase activity assayed by fluorescence polarization

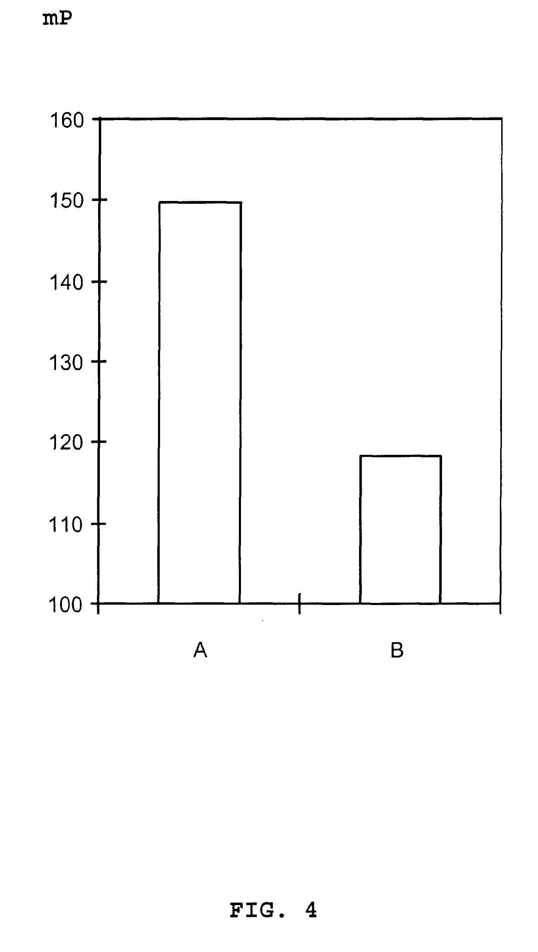

A method for detecting xylanase activity was performed by incubating a BioFeed® Wheat xylanase with a rhodamine labeled xylan and measuring the changes in fluorescence polarization. A decrease in fluorescence polarization of the fluorescent substrate upon hydrolysis by the xylanase was observed. Column B of figure 4 shows a decrease in fluorescence polarization as the labeled xylan is hydrolyzed by the xylanase compared to column A where no xylanase is present. The hydrolysis reaction was conducted by incubating the xylanase with 0,001 % (w/v) rhodamine labeled birch xylan for 30 minutes at 40°C in a pH 6.0 sodium phosphate buffer (100 mM phosphate).

### Example 14; transglutaminase activity assayed by fluorescence polarization

A method for detecting transglutaminase activity was performed by incubating a transglutaminase from *Phytophthora cactorum* with a fluorescently labeled cadaverine as donor and caseine (dephosphorylated) as acceptor and measuring changes in fluorescence polarization. Various fluorescent markers was been tested: fluorescein, dansyl, rhodamine and BODIPY-TR.

**Table 3**

| **Results** | FP +transglutaminase | FP -transglutaminase |
|---|---|---|
| Fluorescein | 65 mP | 25 mP |
| Dansyl | 303 mP | 150 mP |
| Rhodamine | 143 mP | 59 mP |
| BODIPY® -TR | 98 mP | 41 mP |

The results of table 3 shows an increase in fluorescence polarization as the different labeled cadaverines are transferred to the much bigger caseine molecule by the transglutaminase.

The transfer reactions were conducted by incubating the transglutaminase with 16 µM labeled cadaverine and 20.8 µM caseine for 3 hours at 42°C in a pH 7.6 Tris buffer (10 mM Tris, 20 mM CaCl₂ 100 mM NaCl and 4.5 mM dithiothreitol). The FP measurements, however, were performed at room temperature. For dansyl labeled cadaverine excitation and emission wavelengths were 385 nm and 520 nm respectively. For fluorescein labeled cadaverine excitation and emission wavelengths were 485 nm and 525 nm respectively. For rhodamine labeled cadaverine excitation and emission wavelengths were 545 nm and 578 nm respectively. For BODIPY® -TR labeled cadaverine excitation and emission wavelengths were 588 nm and 615 nm respectively. Measurements were obtained by the use of a Perkin Elmer LS50B equipped for fast polarization. Integration time was 1 second.

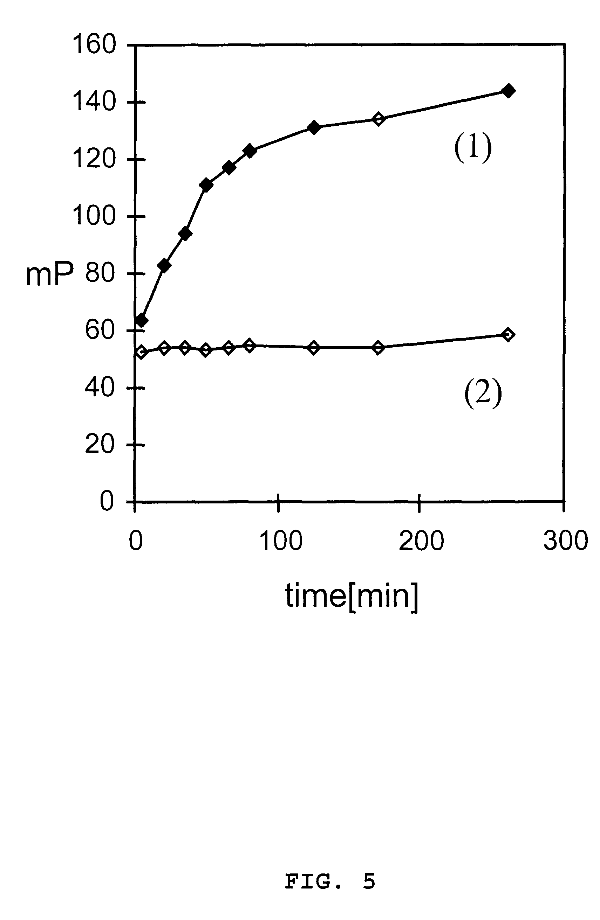

Transglutaminase activity could also be monitored as an increase in fluorescence polarization over time. Figure 5 shows the transfer of rhodamine labeled cadaverine (16 µM) to casein (20,8 µM) by a transglutaminase from *Phytophthora cactorum* over 0-300 minutes (curve 1) compared to the reaction with no transglutaminase present (curve 2). The reactions were conducted at room temperature in a pH 7.6 Tris buffer (10 mM Tris, 20 mM CaCl₂ 100 mM NaCl and 4.5 mM dithiothreitol).

### Example 15; xyloglucan endotransglycosylase activity assayed by fluorescence polarization

A method for detecting xyloglucan endotransglycosylase (XET) activity was performed by incubating a xyloglucan endotransglycosylase with a sulphorhodamine labeled oligo-xyloglucan as donor and xyloglucan as acceptor and measuring changes in fluorescence polarization.

**Table 4**

| **Results** | + XET | - XET |
|---|---|---|
| FP value | 176 mP | 132 mP |

The results of Table 4 show an increase in fluorescence polarization as the labeled oligo-xyloglucan is transferred to the much bigger xyloglucan molecule by the XET enzyme compared to no XET is present. The transfer reaction was conducted by incubating the XET with 2.25 µM sulphohodamin labeled oligo-xyloglucan and 2 g/L xyloglucan over night at ambient temperature in a pH 5,5 succinate buffer (133 mM Na-succinate, 3.8 mM CaCl₂, 1.9 mM L-cystein).

A positive response was obtainable in 2-3 hours by lowering the concentration of oligo-xyloglucan to 0.45 µM (not shown).

### Example 16; pectin methyl esterase activity assayed by fluorescence polarization

A method for detecting Pectin Methyl Esterase (PME) activity was performed by incubating a PME with a fluorescein labeled pectin (77 % degree of methyl esterification of carboxylic groups) and measuring the changes in fluorescence polarization. An increase in fluorescence polarization of the fluorescent pectin upon demethylation by the PME and subsequent cross-linking of demethylated pectin was observed.

**Table 5**

| Results | + PME | - PME |
|---|---|---|
| FP value | 189 mP | 132 mP |

The results of Table 5 shows the increase in fluorescence polarization as the labeled pectin cross-links after demethylation by the PME compared to no PME is present. The demethylation reaction was conducted by incubating a purified PME with 70 µg labeled pectin for 180 minutes at ambient temperature in 200 µl pH 5.0 citrate buffer (100 mM Na-citrate and 2 mM CaCl₂).

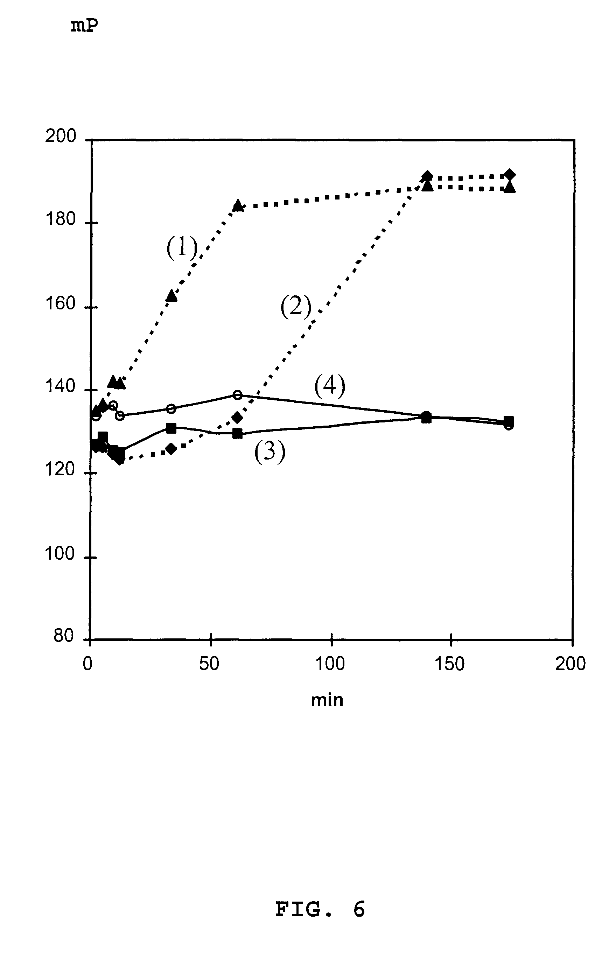

Effects of Ca²⁺ on the reaction kinetics were tested as shown in figure 6. A change of fluorescence polarization could be detected within 30 minutes when Ca²⁺ was added (curve 1). If, however, Ca²⁺ was omitted the reaction kinetic was different and a significant change was seen only after 2.5 hours of incubation (curve 2). For comparison this experiment was repeated omitting the PME, and curve 3 of figure 6 shows the results of adding Ca²⁺, while curve 4 shows the results of omitting Ca²⁺. All reactions were carried out at room temperature in 200 µl pH 5.0 citrate buffer (100 mM Na-citrate) containing 70 µg DE=77% pectin with or without 2 mM added CaCl₂

### Example 17; arabinanase activity assayed by fluorescence polarization

A method for detecting arabinanase activity was performed by incubating an *A. aculeatus* arabinanase with a fluorescein labeled (0.7%) non-debranched arabinan from sugar beet and measuring the changes in fluorescence polarization. A decrease in fluorescence polarization of the labeled arabinan due to hydrolysis or depolymeration by the arabinanase was observed.

**Table 6**

| **Results** | + 5 µL arabinanase | + 0.5 µL arabinanase | + 0.0 µL arabinanase |
|---|---|---|---|
| FP value | 128 | 133 | 141 |

The results of Table 6 shows the decrease in fluorescence polarization as the labeled arabinan is hydrolyzed by respectively 0,5 µL and 5 µL added arabinanase compared to no arabinanase is added. The hydrolysis reaction was conducted by incubating the arabinanase with 170 µg/ml labeled arabinan for 16 hours at 50°C in a pH 5.0 citrate buffer (100 mM Na-citrate and 7 mM NaCl). The reaction volume was 200 µl. FP measurements were performed at ambient temperature.

### Example 18; pectinase activity assayed by fluorescence polarization

A method for detecting pectinase (polygalacturonase I, II and III and a pectin lyase from *A. aculeatus* and a pectate lyase from *B. licheniformis*) activities was performed by incubating various pectinases with two fluorescein labeled pectins with different degrees of methylation or esterification (% DE) and measuring the changes in fluorescence polarization. Decreases in fluorescence polarization of the labeled pectins due to hydrolysis by the pectinases were observed.

Table 7 shows the results of the reactions between different combinations of pectin and pectinase compared to control samples with no pectinase present.

The results verifies that the polygalacturonases, the pectin lyase and the pectate lyase all show the expected reaction pattern. Polygalacturonases and pectate lyase reacted with 3% DE pectin only, while pectin lyase reacted with 77% DE pectin only.

The hydrolysis reactions for polygalacturonases were conducted by incubating the polygalacturonases with respectively 70 µg/ml 77% DE or 35 µg/ml 3% DE labeled pectin for 75 minutes at ambient temperature in a pH 5.0 citrate buffer (100 mM Na-citrate and 2 mM CaCl₂).

The hydrolysis reactions for pectin lyase were conducted by incubating the pectin lyase with respectively 70 µg/ml 77% DE or 35 µg/ml 3% DE labeled pectin for 180 minutes at ambient temperature in a pH 5.0 citrate buffer (100 mM Na-citrate and 2 mM CaCl₂).

The hydrolysis reactions for pectate lyase were conducted by incubating the pectate lyase with respectively 70 µg/ml 77% DE or 35 µg/ml 3% DE labeled pectin for 75 minutes at ambient temperature in a pH 10.0 glycine buffer (50 mM glycine, 100 mM NaCl and 2 mM CaCl₂).

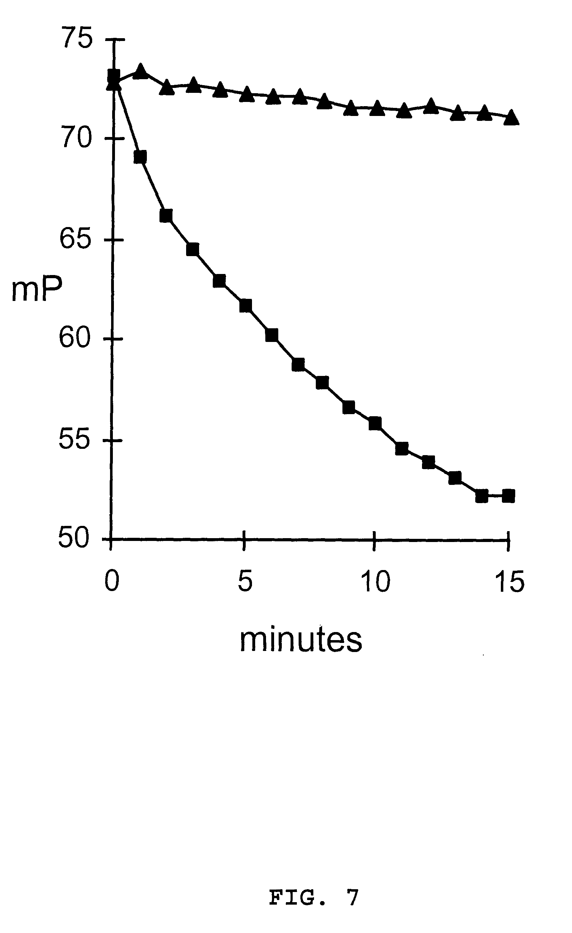

A pectinase expressed by a cloned *E. coli* SJ2 transformant could also be detected by fluorescence polarization. A 50 µl aliquot of clones was taken from a master plate propagated overnight at 37 °C with shaking. The aliquot was mixed in a microtiter well with 150 µl glycine buffer pH 10.0 (4 mM CaCl₂ and 100 mM glycine) containing 6.8 µg fluorescein labeled 3% DE pectin. The fluorescence polarization changes caused by the depolymeration of the pectin was measured over 15 minutes at ambient temperature. The results are shown in figure 7, where FP measurements of a sample containing a transformed *E. coli* SJ2 expressing an uncharacterized pectinase (squares) and a non-transformed *E. coli* SJ2 (triangles) are shown. It can further be derived from figure 7 that the inherent background of *E. coli* SJ2 was very low and did not interfere with the assay.

The method for detection of pectinase activity released from *E. coli* SJ2 present in the microtiter wells is suitable for high through put screening of gene libraries.

**Table 7**

| pectin (% DE) | pectinase | FP + pectinase | FP - pectinase |
|---|---|---|---|
| 3% | Polygalacturonase I | 112 | 201 |
| 77% | Polygalacturonase I | 121 | 129 |
| 3% | Polygalacturonase II | 161 | 195 |
| 77% | Polygalacturonase II | 123 | 120 |
| 3% | Polygalacturonase III | 171 | 205 |
| 77% | Polygalacturonase III | 122 | 129 |
| 3% | Pectate lyase | 67 | 131 |
| 77% | Pectate lyase | 97 | 103 |
| 3% | Pectin lyase | 195 | 197 |
| 77% | Pectin lyase | 105 | 126 |

### Example 19; mannanase activity assayed by fluorescence polarization.

A method for detecting mannanase activity was performed by incubating an *A. aculeatus* mannanase with fluorescein labeled (0.8%) galactomannan from Locus bean gum and measure the changes in fluorescence polarization. A decrease in fluorescence polarisation of the labeled galactomannan due to depolymeration by the mannanase activity was observed.

**Table 8**

| Results | + 1 µl mannanase | + 0 µl mannanase |
|---|---|---|
| FP value | 133 | 156 |

The results shown in Table 8 demonstrate the decrease in fluorescence polarization as labeled galactomannan is depolymerized by addition of 1 µl mannanase compared to no mannanase added. The depolymerization was conducted by incubating the mannanase with 35 µg/ml labeled galactomannan for 2 hours at 40°C in a pH 5.0 citrate buffer (100 mM Na-citrate and 1 mM CaCl₂). The reaction volume was 250 µl and the FP measurements were conducted at ambient temperature.

### Example 20; rhamnogalacturonase activity assayed by fluorescence polarization

A method for detecting rhamnogalacturonase activity was performed by incubating an *A. aculeatus* rhamnogalacturonase with fluorescein labeled (0.1%) rhamnogalacturonan and measure the changes in fluorescence polarization. A decrease in fluorescence polarisation of the labeled rhamnogalacturonan due to depolymeration by the rhamnogalacturonase activity was observed.

**Table 9**

| Results | + 1 µl rhamnogalacturonase | + 0 µl rhamnogalacturonase |
|---|---|---|
| FP value | 80 | 90 |

The results of Table 9 show the decrease in fluorescence polarization as labeled rhamnogalacturonan is depolymerized by addition of 1 µl rhamnogalacturonase compared to no rhamnogalacturonase added. The depolymerization was conducted by incubating the rhamnogalacturonase with 50 µg/ml labeled rhamnogalacturonan for 2 hours at 40°C in a pH 5.0 citrate buffer (100 mM Na-citrate and 1 mM CaCl₂). The reaction volume was 250 µl and the fluorescence polarization measurements were conducted at ambient temperature.

### Example 21; amylase activity assayed by fluorescence polarization by the use of fluorescein labeled amylose as substrate

A method for detecting amylase activity by the use of fluorescently labeled amylose was performed by incubating an *C. thermosulfurogenes* β-amylase or the Novamyl amylase with fluorescein labeled (0.7%) amylose and measure the change of the fluorescence polarization. A decrease in fluorescence polarization of the labeled amylose due to depolymeration by the amylase activity was observed.

**Table 10**

| Results | + 1 µl β-amylase | + 0 µl β-amylase | + 1 µl Novamyl | + 0 µl Novamyl |
|---|---|---|---|---|
| FP value | 188 | 225 | 134 | 225 |

The results of table 10 show the decrease in fluorescence polarization as labeled amylose is depolymerized by addition of 1 µl β-amylase or 1 µl Novamyl compared to no amylase added. The depolymerization was conducted by incubating the amylases with approximately 10 µg/ml labeled amylose for 2 hours at 42°C in a pH 6 buffer (50 mM MES (2-[N-Morpholino]ethanesulfonic acid) and 1 mM CaCl₂). The reaction volume was 250 µl and the fluorescence polarization measurements were conducted at 42°C.

### Example 22; amylase activity assayed by fluorescence polarization by the use of fluorescein labeled amylopectin as substrate

A method for detecting amylase activity by the use of fluorescently labeled amylopectin was performed by incubating an *C. thermosulfurogenes* β-amylase or the Novamyl amylase with fluorescein labeled (0.4%) amylopectin and measure the change of the fluorescence polarization. A decrease in fluorescence polarization of the labeled amylopectin due to depolymeration by the amylase activity was observed.

**Table 11**

| Results | + 1 µl β-amylase | + 0 µl β-amylase | + 1 µl Novamyl | + 0 µl Novamyl |
|---|---|---|---|---|
| FP value | 139 | 184 | 113 | 184 |

The results of Table 11 show the decrease in fluorescence polarization as labeled amylose is depolymerized by addition of 1 µl β-amylase or 1 µl Novamyl compared to no amylase added. The depolymerization was conducted by incubating the amylases with approximately 10 µg/ml labeled amylose for 2 hours at 42°C in a pH 6 MES buffer (50 mM MES (2-[N-Morpholino]ethanesulfonic acid) 1 mM CaCl₂). The reaction volume was 250 µl and the fluorescence polarization measurements were conducted at 42°C.

### Example 23; amylase activity assayed by fluorescence polarization by the use of fluorescein labeled limit dextrins as substrate

A method for detecting amylase activity by the use of fluorescently labeled limit dextrin (DP 15-33) was performed by incubating the Termamyl amylase with fluorescein labeled limit dextrin and measure the change of the fluorescence polarization. A decrease in fluorescence polarization of the labeled limit dextrin due to depolymeration by the amylase activity was observed.

**Table 12**

| Results | + 1 µl Termamyl | + 0 µl Termamyl |
|---|---|---|
| FP value | 168 | 204 |

The results of Table 12 show the decrease in fluorescence polarization as labeled limit dextrin is depolymerized by addition of 10 µl Termamyl compared to no amylase added. The depolymerization was conducted by incubating the amylase with 1 mg/ml labeled limit dextrin for 24 hours at 50 °C in a pH 6.5 MES buffer (50 mM MES (2-[N-Morpholino]ethanesulfonic acid), 1 mM CaCl₂). The fluorescence polarization measurements were conducted at ambient temperature.

### Example 24; cellulase activity assayed by fluorescence polarization

A method for measuring cellulase activity was performed by incubating 20 µl of *Humicola insolens* Cel45 cellulase in various dilutions with 200 µl substrate in a microtiter plate for 1h at 40°C, 400 rpm shaking, and measuring the resulting change in fluorescence polarization. The substrate was 2 mg/ml phosphoric acid swollen cellulose (PASC) which had been covalently labeled with fluorescein. The fluorescence polarization values decrease steadily with increasing enzyme concentration, as shown in table 13 (double determination). FP values of fluroescein-labeled phosphoric acid swollen cellulose after incubation with cellulase, as described. The instrument was calibrated using fluorescein as known standard (35 mP).

**Table 13**

| Cellullase conc. (% of stock) | mP | mP |
|---|---|---|
| 0,0% | 216,67 | 214,58 |
| 1,6% | 189,31 | 192,40 |
| 3,1% | 175,74 | 181,16 |
| 6,3% | 160,67 | 159,82 |
| 12,5% | 146,20 | 154,92 |
| 25,0% | 132,09 | 138,31 |
| 50,0% | 127,23 | 124,54 |
| 100,0% | 123,00 | 125,04 |

### Example 25; high through-put screening of genomic libraries derived from a strain of Bacillus (identical to B. licheniformis, CA63, DSM 9552) and a strain of Bacillus (identical to B. clausii, NCIMB 10309, DSM 8716) formerly named B. lentus for nucleotide sequences encoding for pectinase

A schematic representation of the screening procedure is shown in figure 1. The content of a vial with SJ2 *E. coli* host cells transformed with a library was inoculated into 10 ml of LB medium and incubated for 40 minutes at 37°C. Subsequently, the 10 ml of cells were diluted in cold LB medium with chloramphenicol (10 µg/ml) to a final concentration of cells corresponding to 0.5-1 cell/300 µl medium.

Diluted transformants and clones thereof were kept on ice while aliquots of 300 µl/well were transferred into NUNC microtiter plates using a Micro droplet instrument. Typically 40-50 plates were sufficient for screening one bacterial library. The plates were placed in a tightly closed box in order to avoid evaporation of medium from the wells and placed on a shaker for approximately 70 hours with shaking (approx. 175 rpm) at 37°C. Black microtiter plates were filled with 150 µl of a solution of 34 µg/ml fluorescein-5-thiosemicarbazide labeled pectin with different degree of methylation and 4 mM CaCl₂ in a buffer supporting the wanted pH (see Table 14). Subsequently, 50 µl aliquots of the transformant/clone suspensions were pipetted from the master plates into the black assay plates using an automated pipetting station. The assay plates were left at room temperature (in the dark) for approximately 150 min and subsequently read by a fluorescence polarization reader (FPM-2, Jolley Consulting, IL). Positive hits were scored as a lowering of the fluorescence polarization value. Non-reacted pectins had a mP value of 70-200 depending on the source of the pectin and the degree of methylation, while pectins degraded by pectinase yielded a value of 60-130 mP.

Libraries from the two different nucleotide sources were screened under different conditions and with different number of transformants or clones per well at the separation step (figure 1 picture 4). Table 14 shows the nucleotide sources for libraries screened for pectinases, the used substrates, and the number of positive hits obtained. The term Clones/well was calculated according to the statistical standard equation for Poison-distribution as -logₑ(frequency of empty wells), where (frequency of empty wells) = (empty wells)/(total number of wells). Further Table 14 shows that positive hits were found for all pectin substrates, and subsequent sequence analyses revealed that nucleotide sequences encoding for at least two pectate lyases (*B.* *licheniformis* and *B. clausii*) and one pectin lyase (*B. clausii)* were found. The procedures also demonstrated that multiple nucleotide sequences encoding for enzymes can be screened simultaneously by pipetting the library into different microtiter plates with various substrates as done with the *B. clausii* library.

**Table 14**

| Screening | Nucleotide source | Substrate | No of clones /well | Total no of clones | Positive hits |
|---|---|---|---|---|---|
| **1** | *B. licheniformis* | 3% pectin, pH 10 | 6.0 | 12672 | 2 |
| **2** | *B. licheniformis* | 3% pectin, pH 10 | 3.8 | 16416 | 3 |
| **3** | *B. clausii* | 3% pectin, pH 10 | >8 | >16128 | 2 |
| **4** | *B. clausii* | 35% pectin, pH 10 | 0.6 | 2304 | 3 |
| **5a** | *B. clausii* | 3% pectin, pH 10 | 2.2 | 9504 | 4 |
| **5b** | *B. clausii* | 77% pectin, pH 10 | 2.2 | 9504 | 3 |

### Example 26; The number of hits as a function of the number of different transformed host cells per microtiter plate well

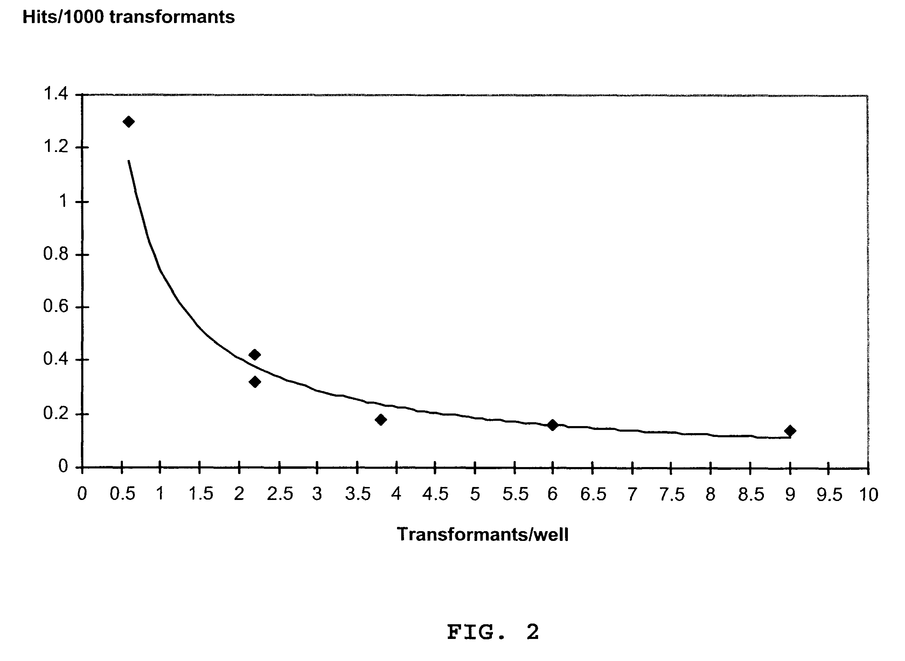

In order to investigate the number of hits as a function of the number of different transformants or clones, transformed with the pSJ1678 plasmid, to be inoculated into each well, (figure 1 picture 4), the transformants and clones (figure 1 picture 3) were diluted to a concentration enabling transfer of 300 µL aliquots containing a range from 0.5 transformant or clone per aliquot to more than 8 transformants and/or clones per well. Table 15 and figure 2 shows that the number of transformants and/or clones per well is an important parameter for pectinases. In Table 15 the number of positive hits/1000 transformants are shown along with the number of transformants or clones per well. Figure 2 shows the same information in a graphical format. The diamonds in figure 2 indicate hits per 1000 screened transformants. The transformants/well term represents the average number of transformants or clones per well, based on the assumption that the distribution of transformants or clones the wells follow the Poison distribution. Thus the average number can be calculated according to the standard statistical equation: average = -logₑ(number of empty wells)/(total number of wells).

The results were obtained for *B. licheniformis* with fluorescein labeled 3 %DE pectin at the conditions given in Example 1. The result were valid for *B. clausii* and other %DE pectins as well.

Without being bound to the theory it is presently contemplated that the importance of restricting the number of different transformed host cells per well in the separation step is likely due to growth inhibition of "positive hit" clones by clones not expressing a biological compound able of reacting with the chosen fluorescent substance(s).

**Table 15**

| Screening | No of clones/ well | Total no of clones | Positive hits | Hits/1000 |
|---|---|---|---|---|
| **1** | 6.0 | 12672 | 2 | 0.16 |
| **2** | 3.8 | 16416 | 3 | 0.18 |
| **3** | >8 | >16128 | 2 | 0.14 |
| **4** | 0.6 | 2304 | 3 | 1.3 |
| **5a** | 2.2 | 9504 | 4 | 0.42 |
| **5b** | 2.2 | 9504 | 3 | 0.32 |

### Example 27; high through-put screening of genomic library derived from a strain of Bacillus sp. (DSM 12708) for nucleotide sequences encoding for amylases by the use of fluorecein labeled starch

The content of a vial with DH10B *E. coli* host cells transformed with a library cloned in the pZErO-2 vector was inoculated into 2.9 litre of LB medium supplemented with kanamycin (10 µg/ml). The LB medium with the inoculated library was kept on ice while aliquots of 300 µl/well were transferred into NUNC 96 wells microtiter plates using a Micro droplet instrument giving a total of 100 plates. In average 1.5 clones were distributed into each well making up a total of approximately 14400 clones. The plates were placed in a tightly closed box in order to avoid evaporation of medium from the wells and placed on a shaker for approximately 48 hours with shaking (approximately 200 rpm) at 37°C.

50 µl aliquots of the transformant/clone suspensions were pipetted from the master plates into black 96 wells assay plates using an automated pipetting station. Subsequently, by use of a Micro droplet instrument 220 µl of a solution of 30 µg/ml fluorescein labeled starch (Sigma), 50 mM MES (2-[N-Morpholino]ethanesulfonic acid) pH 6, 3 mM CaCl₂ and 1 mM NaCl₂ were added to each well. The microtiter plates were sealed with a sealing foil (NUNC, Denmark) and placed in an incubator at 60°C for 120 minutes. Prior to removal of the sealing foil the plates were cooled at room temperature for approximately 60 minutes. The plates were read by a fluorescence polarization reader (Polarstar, BMG, Germany) at ambient temperature and positive hits were scored as lowering of the fluorescence polarization value. Positive hits were defined as polarization values lower than the average polarization value of each microtiter plate minus 3.5 times the standard derivation of the 96 measurements derived from each microtiter plate. The average standard variation was approximately 2.2 mP units. A total of 10 confirmed positive hits (amylase expressing clones) were identified which corresponds to 1 hit in 1440 clones screened. DNA sequencing of the clones demonstrated that at least 2 different amylase encoding genes had been cloned.

### Example 28; high through-put screening of genomic library derived from a strain of Bacillus sp. (DSM 12709) for nucleotide sequences encoding for amylases by the use of fluorescein labeled amylose

The content of a vial with DH10B *E. coli* host cells transformed with a library cloned in the pZErO-2 vector was inoculated into 2.9 litre of LB medium supplemented with kanamycin (10 µg/ml). The LB medium with the inoculated library was kept on ice while aliquots of 300 µl/well were transferred into NUNC 96 wells microtiter plates using a Micro droplet instrument giving a total of 100 plates. In average 0.4 clones were distributed into each well making up a total of approximately 3840 clones. The plates were placed in a tightly closed box in order to avoid evaporation of medium from the wells and placed on a shaker for approximately 48 hours with shaking (approximately 200 rpm) at 37°C.

50 µl aliquots of the transformant/clone suspensions was pipetted from the master plates into black 96 wells assay plates using an automated pipetting station. Subsequently, by use of a Micro droplet instrument 220 µl of a solution of 10 µg/ml fluorescein labeled amylose, 50 mM MES (2-[N-Morpholino]ethanesulfonic acid) pH 6, and 1 mM CaCl₂ were added to each well. The microtiter plates were sealed with a sealing foil (NUNC, Denmark) and placed in an incubator at 60°C for 120 minutes. Prior to removal of the sealing foil the plates were cooled at room temperature for approximately 60 minutes. The microtiter plates were read by a fluorescence polarization reader (Polarstar, BMG, Germany) at ambient temperature and positive hits were scored as lowering of the fluorescence polarization value. Positive hits were defined as polarization values lower than the average polarization value of each microtiter plate minus 3.5 times the standard derivation of the 96 measurements derived from each microtiter plate. The average standard variation was 5.6 mP units. A total of 3 confirmed positive hits (amylase expressing clones) were found which corresponds to 1 hit in 1280 clones screened.

### Example 29; high through-put screening of genomic library derived from a strain of Bacillus sp. (DSM 12682) for nucleotide sequences encoding for cellulase by the use of fluorescein labeled PASC

The assay method of example 24 was also used to assay for cellulase activity in a *Bacillus subtilis* clone, DSM 12682. DSM 12682 is a *B. subtilis* 168 with deleted *apr, npr, amyE* and *celA* genes and which had been transformed with a pUB110 vector containing the *Cel*5a gene from *B. agaradhaerens* (GenBank AF067428) in fusion with the *amyL* signal peptide of *B. licheniformis* and the *amyL* promoter. DSM 12682 was incubated overnight in TY media at 37°C at 250 rpm. 20 µl of DSM 12682 supernatant was incubated with 200 µl substrate under the conditions described in example 24. A fluorescence polarization value of 129 mP was recorded, versus 214 mP for the control strain with no plasmid. This experiment demonstrates that the presence of cells in the assay mix does not hamper the quality of the assay.

### Example 30; screening for enzymatic activities in wild type isolates by fluorescence polarization

Two wild type isolates, Bacillus sp. (DSM 12663) and Bacillus sp. (DSM 12664), were assayed for pectinase activity at 90°C at pH 9. The isolates were grown in BP-X medium (pH 9.7) for 5 days at 30°C. After out-growth of the culture 50 µl was mixed with 250 µl preheated assay buffer (35 µg/ml fluorescein labeled pectin DE 3%, 50 mM Bicine pH 9 (N,N-bis[2-Hydroxyethyl]glycine) and 3 mM CaCl₂). The assay was conducted in Eppendorf tubes incubated at 90°C for 2 hours. Subsequently to cooling the assay mix was transferred into black microtiter plates and read by a fluorescence polarisation reader (Polarstar, BMG, Germany). The fluorescence polarisation analyses of the 2 wild type isolates is depicted in Table 16.

**Table 16**

| **Isolate** | **mP** |
|---|---|
| Bacillus. sp. DSM 12663 | 125 |
| Bacillus. sp. DSM 12664 | 177 |
| Medium without cells | 195 |

As can be seen from Table 16 the Bacillus (DSM 12663) isolate had a lower fluorescence polarization value than the Bacillus (DSM 12664) isolate and the growth medium without cells. It was later made it likely that Bacillus (DSM 12663) did encode a pectinase as a chromosomal library from a phylogenetic related species (most likely the same species) was constructed and screened for the presence of nucleotide sequences encoding pectinase activity. A nucleotide sequence encoding a pectinase was isolated. This shows that enzymatic activities assayed in wild type isolates by fluorescence polarization may later be cloned.

### References

Ausubel, F. M. et al. *(eds.) Current protocols in Molecular Biology,* John Wiley and Sons, 1995.
Christgau, S., Kofoed, L.V., Halkier, T., Andersen, L.N., Hockauf, M., Dorreich, K., Dalbøge, H., and Kauppinen, S., *"Pectin methyl esterase from Aspergillus aculeatus: expression cloning in yeast and characterisation of the recombinant enzyme",* Biochem J., 319, pp 705-712, 1996.
Diderichsen, B., Wedsted, U., Hedegaard, L., Jensen, B. R., Sjøholm, C., *"Cloning of aldB, which encodes alpha-acetolactate decarboxylase, an exoenzyme from Bacillus brevis",* J. Bacteriol., 172, pp 4315-4321, 1990.
Dretzen, G., Bellard, M., Sassone-Corsi, P., Chambon, P., *"A reliable method for the recovery of DNA fragments from agarose and acryla-mide gels",* Anal. Biochem., 112, pp 295-298, 1981.
Fry, S., Smith, R.C., Renwick, K.F., Martin, D.J., Hodge, S.K., and Matthews, K.J., *"Xyloglucan endotransglycosylase, a new wall-loosening enzyme activity from plants",* Biochem. J., 282, pp 821-828, 1992.
Fry, S., *"Novel dot-blot assay for glycosyltransferases and glycosylhydrolases: optimization for xyloglucan endotrans-glycosylase (XET) activity",* The Plant Journal, 11, pp 1141-1150, 1997.
Harwood, C. R., and Cutting, S. M. (eds.), *"Molecular Biological Methods for Bacillus",* John Wiley and Sons, 1990.
Pitcher, D. G., Saunders, N. A., Owen, R. J., *"Rapid extraction of bacterial genomic DNA with guanidium thiocyanate",* Lett. Appl. Microbiol., 8, pp 151-156, 1989.
Sambrook et al., *"Molecular cloning: A laboratory manual",* Cold Spring Harbor lab., Cold Spring Harbor, NY. (1989) Poulsen L.K., Refn A., Molin S. and Andersson P., *Topographic analysis of the toxic Gef protein from Escherichia coli,* Molecular Microbiology, 5(7), pp.1627-1637, 1991
Eisenstadt E., Carlton B.C. and Brown B.J., *Gene mutation,* Methods for general and molecular bacteriology, pp. 297-316, Eds: Gerhardt P., Murray R.G.E., Wood W.A. and Krieg N.R., ASM, 1994.
Stemmer, Proc. Natl. Acad. Sci. USA, 91, pp. 10747-10751, 1994.
Stemmer, Nature, 370, pp. 389- 391, 1994.
Christiansen L.C., Schou S., Nygaard P. and Saxild H.H., *Xanthine metabolism in Bacillus subtilis: Characterization of the xpt-pbuX operon and evidence for purine and nitrogen controlled expression of genes involved in xanthine salvage and catabolism,* Journal of Bacteriology, 179(8), pp 2540-2550, 1997.
Stoss O., Mogk A. and Schumann W., *Integrative vector for constructing single copy translational fusions between regulatory regions of Bacillus subtilis and the bgaB reporter gene encoding a heat stable beta-galactosidase,* FEMS Microbiology Letters, 150(1), pp 49-54, 1997.
Checovich W., Bolger R. & Burke T., *Fluorescence polarization - a new tool for cell* and *molecular biology,* Nature, 375, 254-256, 1995).
Ohuchi, S. et al.; *In vitro method for generation of protein libraries using PCR amplification of a single DNA molecule and coupled transcription/translation;* Nucleic Acid research, 1998, vol. 26. No. 19, pp. 4339-4346.
Simonen M., Palva I.; *Protein Secretion in bacillus species;* Microbial Reviews; 1993; vol. 57; pp. 109-137.
Gotz F. et al.; *Staphylococcal lipases;* Molecular characterisation, secretion and processing; 1998; vol. 93; pp. 15-25
Pugsley A. P. et al.; *Analysis of the subcellular location of pullulanase produced by Escherichia coli carrying the pulA gene from Klebsiella pneumoniae strain UNF5023;* Molecular Microbiology; 1990; vol. 4; pp. 59-72.
Gerhardt P. et al.; *Methods for general and molecular biology;* American Society for Microbiology; 1994; Eds: Gerhardt P., Murray R.G.E., Wood W.A., Krieg N.R.
Ellman J., Mendel D., Anthony-Cahill S.J., Noren C.J. and Schultz P.G., *Methods in Enzymol.* 1991; vol. 202; pp. 301-337.
Schülein M.; J. Biotechnology; 1997; vol. 57, pp. 71-81.
Shen et al.; *Purification and characterization of an novel thermostabel beta-amylase from Clostridium thermosulphurogenes;* Biochem. J.; 1988; vol. 254; pp. 835-840.
Tada S., Iimura Y., Gomi K., Takahashi K., Hara S., Yoshizawa K.; *Cloning and nucleotide sequence of the genomic Taka-amylase A gene of Aspergillus oryzae;* Agric. Biol. Chem.; 1989; vol. 53; pp.593-599.
Arndt D.J. et al.; *Studies of cellular differentiation by automated cell separation: Two model systems: Friend virus-transformed cells and Hydra attenuata;* J. Histochemistry and Cytochemistry; 1976; vol. 24; no. 1; pp. 332-347
Lindmo T. and Steen H.B.; *Flow cytometric measurement of the polarization of fluorescence from intracellular fluorescence in mammalian cells;* Biophysical Journal; 1977; vol. 18; pp. 173-187.

## Claims

1. A method for determination of transferase activity comprising measuring changes in fluorescence polarization of a fluorescently labeled substance upon transfer to a second compound by the transferase.

2. The method according to claim 1, wherein the transferase is a transglutaminase, the substance is a fluorescently labeled peptide and the second compound is a protein.

3. The method according to claim 1, wherein the transferase is a xyloglucan endotransglycosylase, the substance is a fluorescently labeled oligo xyloglucan and the second compound is a xyloglucan.

4. The method according to claims 1-3, wherein the enzyme activity is determined in a fermentation broth.

5. The method according to claims 1-3, wherein the enzyme activity is determined in a purified or formulated enzyme product.
